# EUROPEAN PATENT SPECIFICATION

(11) **EP 3 290 008 B1**
(45) Date of publication and mention of the grant of the patent: **15.09.2021**
(21) Application number: 16785981.8
(22) Date of filing: 29.04.2016
(51) Int. Cl.: A61F 2/95, A61F 2/90, A61F 2/966

(54) **SUPPORT AND DRUG DELIVERY DEVICE**
UNTERSTÜTZUNGS- UND WIRKSTOFFFREISETZUNGSVORRICHTUNG
SUPPORT ET DISPOSITIF D'ADMINISTRATION DE MÉDICAMENT

(30) Priority: 30.04.2015 CN 201510219273
(43) Date of publication of application: 07.03.2018
(73) Proprietor: Shanghai VasoLutions MedTech Co., Ltd., Shanghai 201318 (CN)
(72) Inventor: LI, Zhonghua, Shanghai 201318 (CN); CHEN, Zhong, Shanghai 201318 (CN); ZHANG, Linlin, Shanghai 201318 (CN); GUO, Fang, Shanghai 201318 (CN); HAN, Jianchao, Shanghai 201318 (CN); ZHANG, Peng, Shanghai 201318 (CN); MIAO, Zhenghua, Shanghai 201318 (CN)
(74) Representative: Patentship Patentanwaltsgesellschaft mbH
(86) International application number: PCT/CN2016/080754
(87) International publication number: WO 2016/173553

(56) References cited:
- CN-A- 101 869 514
- CN-A- 101 933 855
- CN-U- 202 409 217
- DE-A1-102010 027 123
- US-A1- 2006 190 075
- US-A1- 2008 262 592
- US-A1- 2008 281 403
- US-A1- 2010 280 592
- US-A1- 2011 009 950
- US-A1- 2011 106 234
- US-A1- 2012 158 122
- US-B1- 6 176 873

## Description

### TECHNICAL FIELD

The present invention relates to implantable medical devices and, in particular, to an implantable drug-eluting stent (DES) and a corresponding drug delivery device.

### BACKGROUND

Percutaneous coronary intervention (PCI) is a technique to reconstruct the coronary artery blood flow by delivering a balloon catheter or other related device through percutaneous puncture and removing coronary artery stenosis or occlusion. Commonly-used existing interventional devices include bare balloons, cutting balloons, bare stents, drug-eluting stents, etc. Although these devices have been proven to be beneficial for the treatment of stenotic lesions, they are all suffering from some deficiencies.

A drug-eluting stent (DES) is coated on its surface with a drug in a polymer matrix, which provides a local slow drug-release system. After the stent is implanted into a vessel, the drug will be slowly released in a time period ranging from several weeks to several months. This, coupled with the support by the stent, can effectively treat the stenosis or occlusion in the vessel. Therefore, DESs are one of the most commonly-used and most effective existing interventional devices. However, they are still associated with a number of issues such as, for example, local, chronic inflammatory responses possibly caused by the polymer, necessitation of a long-term antiplatelet therapy due to the long-term presence of the stent, non-uniform distribution of the drug in the coating, and inability to allow the implantation of a new stent even when thrombosis occurs in the stent.

In recent years, there has been developed a novel interventional device, called a drug-eluting balloon (DEB). DEB technique is an improved balloon angioplasty in which a balloon coated with a drug on its outer surface is delivered by a catheter to the site of a target lesion such as stenosis or obstruction, followed by expansion of the balloon within a short space of time (0-5 min) which causes the drug coated thereon to be released at the lesion site. This achieves expansion of the blood vessel and treatment of the lesion. In addition, when restenosis or re-occlusion occurs at the treated vascular lesion, a new DEB treatment is allowed to deal with it. However, in practice, the DEB technique is faced with such a challenge that it is difficult to ensure both that the drug adheres to the balloon with sufficient strength to prevent significant loss of the drug during the delivery and that the drug is released at the lesion site during the expansion of the balloon at a sufficient amount to exert a desired therapeutic effect.

Presently, in the field of research and development of drug-coated stents, in order to reduce or avoid the side effects of long-term presence of polymers on the human body, most researchers choose to employ biodegradable polymers or not use polymers at all. However, the issues arising from the long-term presence of stents in the human body remain unresolved. While use of biodegradable stents is one of the focuses of current research efforts, such biodegradable stents are limited in certain applications due to their higher proneness to rupture and weaker support than metal stents. On the other hand, in the field of research and development of drug-coated balloons, most researchers are seeking to solve the challenge they are faced with through design of special balloon structures or development of drug carriers with special capabilities. However, there has not been significant progress to the date in this task possibly due to high difficulty. Extraordinary results will be obtained from merging the support capabilities of stents with fast release capabilities of drug-coated balloons.

Document US 2012/158122 A1 discloses a removable stent and method of production.

Document US 2011/0106234 A1 discloses interluminal medical treatment devices and methods.

### SUMMARY OF THE INVENTION

It is an objective of the present invention to provide a stent and a drug delivery device. When implanted in the human body, the drug-carrying stent provides fast drug release performance comparable to that of a drug-eluting balloon (DEB) while not compromising its support performance as a stent. The stent is recoverable and can thus avoid the issues arising from its long-term presence in the body.

To this end, in a first aspect of the present invention, there is provided a stent including a tubular main body. The main body has a first end provided with a connecting structure configured for connection with a recovery mechanism, and wherein a drug is applied on a surface of the main body.

In the stent, the main body may be a mesh structure having a mesh density gradually increasing from the first end to a second end of the main body, and the connecting structure is provided at the first end of the main body where the mesh density is lowest.

In the stent, the connecting structure may include one or more connecting terminals.

In the stent, the connecting structure may include at least two pairs of opposing connecting terminals connected to each other by a suture so that a cross is formed.

In the stent, an additional connecting terminal may be provided at an intersection of the cross.

In the stent, the main body has an inner or outer surface attached with a cover layer, the cover layer may be coated with the drug.

In the stent, the cover layer is a bioabsorbable film.

In the stent, the tubular body may define micropores or microgrooves in which the drug is contained.

In the stent, the drug may be coated on the surface of the main body.

In the stent, the drug may include one or more of acemetacin, aescine, aminopterin, antimycoin, arsenic trioxide, aristolochic acid, aspirin, berberine, bilobol, rapamycin and derivatives thereof, paclitaxel, docetaxel, antibiotics and hormones.

In the stent, the surface of the main body may be further applied with a hydrophilic additive which is a water-soluble organic substance containing one or more functional groups selected from the group consisting of hydroxyl groups (-OH), amino groups (-NH₂), amide groups (-CONH-), sulfonic acid groups (-SO₃H) and carboxyl groups (-COOH).

In a second aspect of the present invention, there is provided a drug delivery device including a stent as defined above and a recovery mechanism. The recovery mechanism includes a sheath and a recovery wire that passes through the sheath and has one end connected to the connecting structure of the stent. The sheath is adapted to receive the stent when the stent is in a contracted configuration.

In the drug delivery device, the one end of the recovery wire may be fixedly connected to the connecting structure of the stent.

In the drug delivery device, the connecting structure may include a plurality of connecting terminals, wherein one end of the recovery wire defines a branched structure and each branch of the branched structure is configured to connect a corresponding one of the plurality of connecting terminals of the connecting structure.

Compared with the conventional drug-carrying stents, the stent and drug delivery device of the present invention can be recovered after the carried drug is released. This prevents the disadvantages arising from long-term presence of the stent in the human body. Compared with the conventional drug-coated balloons, the recoverable stent and drug delivery device of the present invention allows drug to be released over a prolonged period of time and better absorbed without the occurrence of blood flow blockage. Further, the stent can support the vessel to prevent its contraction for a short period of time.

### BRIEF DESCRIPTION OF THE DRAWINGS

Figs. 1 to 2 schematically illustrate a first embodiment of the present invention.
Figs. 3 to 8 schematically illustrate a second embodiment of the present invention.
Fig. 9 is a schematic illustration of a recovery wire according to the second embodiment of the present invention.
Figs. 10 to 15 schematically illustrate a third embodiment of the present invention.
Figs. 16 to 17 schematically illustrate a fourth embodiment of the present invention.
Figs. 18 to 22 show variants of connection between a stent and the recovery wire.

### DETAILED DESCRIPTION

The present invention will be described in greater detail below with reference to the accompanying drawings and several specific embodiments.

### EMBODIMENT 1

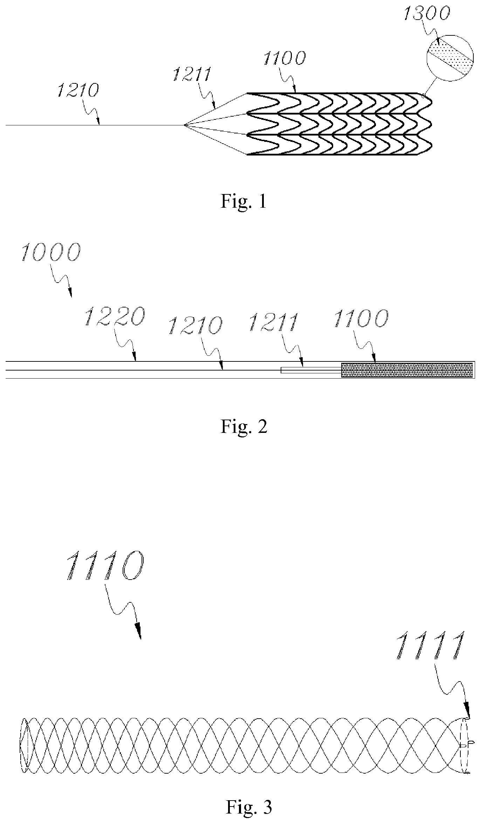

Referring to Fig. 1, a stent 1100 according to this embodiment includes a tubular body that is braided from a plurality of W-shaped nickel-titanium (NiTi) alloy wires. The braid density of the tubular body gradually increases along its length from one end to the other. A connecting structure is defined at the end of the stent 1100 where the density is lowest. As can be seen from Fig. 1, the connecting structure includes a number of connecting terminals.

75 mg of paclitaxel and 45 mg of polyurea were dissolved in 2-4 ml of an acetone/ethanol mixture to obtain a solution. The solution was sprayed onto the surface of the tubular body and dried to obtain a drug-containing coating 1300, thereby forming a drug-carrying stent.

The stent 1100 according to this embodiment is a recoverable stent for use with a recovery mechanism. In this embodiment, the recovery mechanism includes a sheath 1220 (Fig. 2) and a recovery wire 1210. The recovery wire 1210 has a branched structure 1211 at one end thereof. Preferably, the number of the branches of the branched structure 1211 is equal to the number of connecting terminals of the connecting structure of the stent 1100, so that each of the branches of the branched structure 1211 can be connected to a corresponding one of the connecting terminals to accomplish the pre-connection between the recovery wire 1210 and the stent 1100. The stent 1100 and the recovery mechanism constitute a drug delivery device 1000.

The stent 1100 is be compressible. As shown in Fig. 2, the stent 1100 can be contracted into the sheath 1220. After the external forces are removed, the stent can recover its original shape by itself or with the aid of a balloon.

During use in surgery, after the drug delivery device 1000 has reached the site of a target lesion, the recovery wire 1210 is fixed and the sheath 1220 is retracted, so that the stent 1100 is released from the sheath 1220 at the site of the target lesion. The sheath 1220 is subsequently retrieved from the body.

In this embodiment, as the stent 1100 is undetachably connected with the recovery wire 1210, the recovery wire 1210 stays with the stent 1100 in the human body (e.g., for 10 hours).

In order to recover the stent 1100, the sheath 1220 is guided along the recovery wire 1210 to a predetermined location. Subsequently, the recovery wire 1210 is fixed, and the sheath 1220 is advanced toward the stent 1100, until the stent 1100 is gradually contracted and loaded into the sheath 1220. As the part of the stent around the connecting structure is lowest in braid density and hence in radial resistance, it is ensured that the stent can be easily loaded into the sheath 1220 during the retrieval.

After that, the entire drug delivery device 1000 is retrieved from the human body.

### EMBODIMENT 2

Referring to Fig. 3, a stent 1110 according to this embodiment includes a tubular body braided from four NiTi wire coils. With similarity to Embodiment 1, the braid density of the tubular body gradually increases along its length from one end to the other. A connecting structure 1111 with four lug rings is defined at the end where the density is lowest.

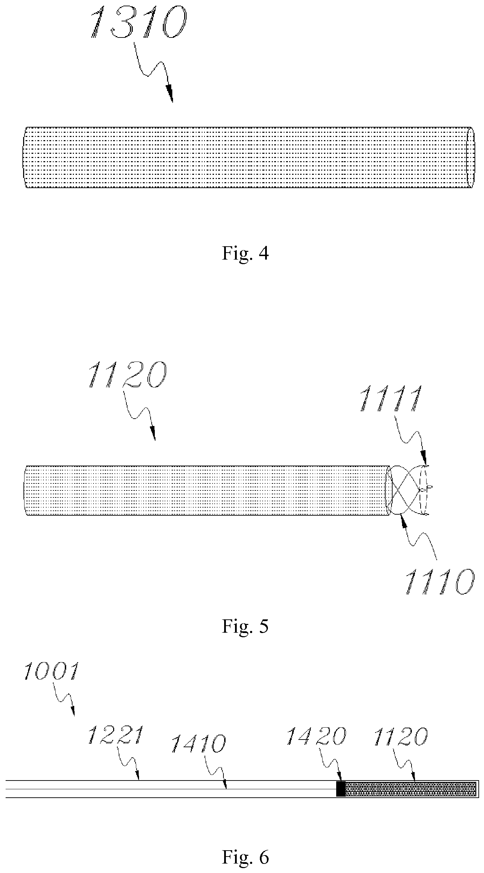

300 mg of sodium alginate and 20 mg of chitosan were dissolved in 10ml of purified water. The solution was heated to 50 °C, added with 0.1ml of glycerol, stirred for 2 hours to achieve homogeneity and vacuum degassed. A layer of Tween 80 was applied on a glass plate, and an ethanol solution of paclitaxel was then uniformly sprayed thereon. After 30 min of maintenance, a degassing liquid was poured onto the glass plate and cast into a film. The glass plate was subsequently dried in an oven at 50 °C for 4 hours, and the film was peeled off as a drug-containing bioabsorbable film 1310, as shown in Fig. 4.

The film 1310 is electrostatically bonded to the outer surface of the stent 1110 to form a stent-graft 1120, as shown in Fig. 5, with the connecting structure 1111 being not covered by the film 1310.

Referring to Fig. 6, the stent-graft 1120 is contracted into a sheath 1221 of a catheter in which a push rod 1410 and a push-out ring 1420 in connection with the push rod 1410 are disposed, thereby forming a drug delivery device 1001.

During use in surgery, the drug delivery device 1001 is delivered to the site of a target lesion, followed by fixation of the push rod 1410 and retraction of the sheath 1221. Under the action of the push-out ring 1420, the stent-graft 1120 is pushed out, i.e., the stent-graft 1120 is released from the sheath 1221 at the site of the target lesion. After that, the sheath 1221, the push rod 1410 and the push-out ring 1420 are retrieved from the human body.

As the film 1310 is bioabsorbable, it will immediately adhere to the vessel wall as soon as it comes into contact with the vessel wall and thereby disengage from the stent 1110.

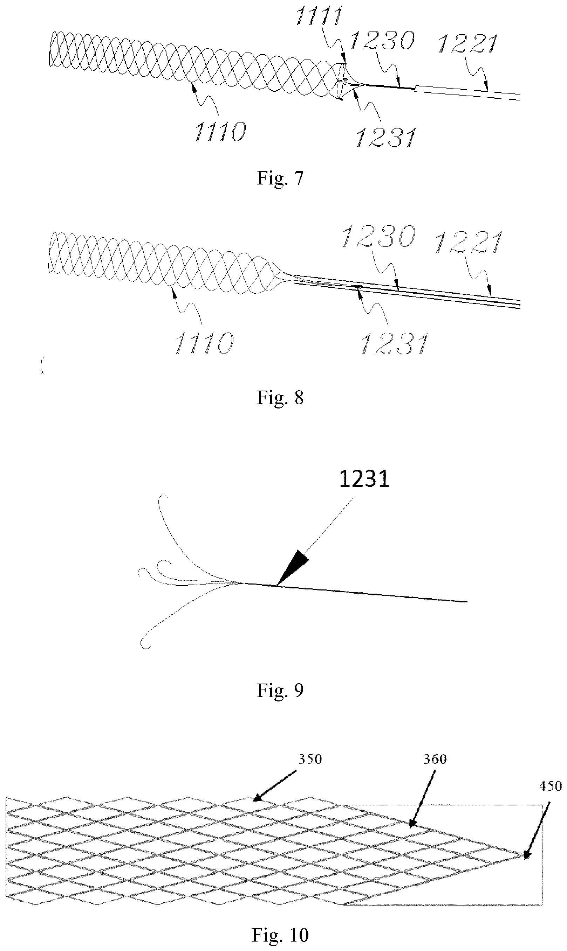

As shown in Fig. 7, in order to recover the stent 1100, a recovery wire 1230, together with the sheath 1221, is guided to the least dense end of the stent 1110. The sheath 1221 is then fixed and the recovery wire 1230 is advanced forward until a branched structure 1231 defined at one end of the recovery wire 1230 is connected to the connecting structure 1111 of the stent 1110. Reference can be made to Fig. 9 for structural details of the branched structure 1231. Each branch of the branched structure has a hook end which can be firmly coupled to a corresponding one of the lug rings of the connecting structure 1111.

Subsequently, the recovery wire 1230 is fixed and the sheath 1221 is pushed forward so that the stent 1110 is gradually contracted and loaded into the sheath 1221, as shown in Fig. 8. Finally, all the components are retrieved from the human body.

### EMBODIMENT 3

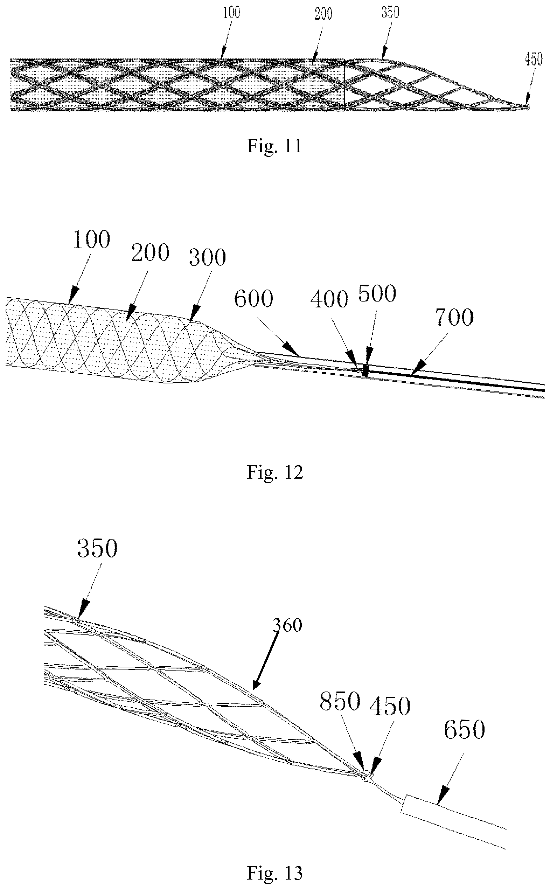

Referring to Figs. 10 and 11, a stent including a main body 350 and a connecting structure 450 is fabricated by laser cutting and thermally expanding a NiTi tube. The main body 350 is a mesh structure. The stent further includes a wedge-shaped mesh transition section 360 between the main body 350 and the connecting structure 450. The wedge-shaped transition section 360 has a mesh density gradually increasing along its length and can facilitate the compression and reception of the stent into a catheter.

Referring to Fig. 11, a latex cover layer 100 is sutured onto the stent so that it is fixed inside the stent but does not cover the connecting structure 450 or the wedge-shaped transition section 360.

75 mg of paclitaxel and 45 mg of polyurea were dissolved in 2-4 ml of an acetone/ethanol mixture to obtain a solution, the solution was sprayed onto the surface of the cover layer and dried to obtain a stent-graft with a drug-containing coating 200.

With additional reference to Fig. 12, the stent is chilled and compressed into a catheter 600, a push rod 700 is disposed in the catheter 600. The push rod 700 has a push-out head 500 at its one end proximal to the stent and is connected at the other end to connector of a stent delivery device. After the stent delivery device has delivered the stent to the site of a target lesion, it is slowly retracted with the push-out head 500 at the leading end of the push rod 700 bearing against the stent until the stent is released from the catheter 600 at the site of the target lesion.

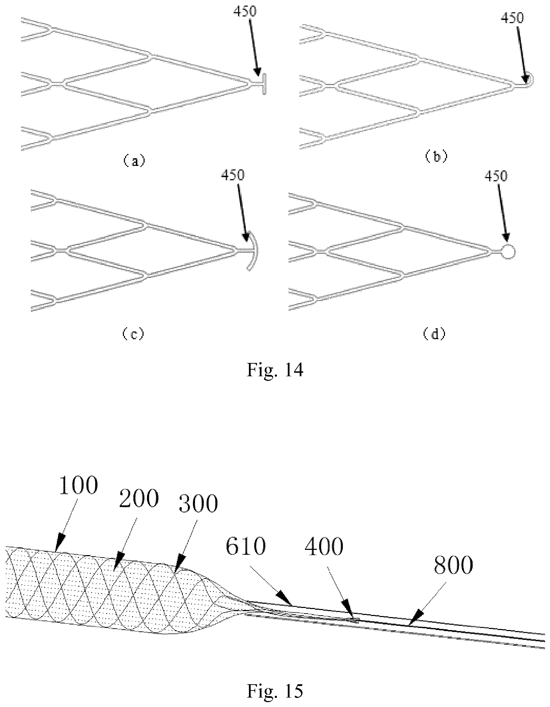

As shown in Fig. 13, in this embodiment, the stent is detachable from a recovery wire 850. The delivery device and the push rod are retrieved from the human body, while the stent is left in the body. One end of the recovery wire 850 engages the connecting structure 450. The connecting structure 450 is defined at one end of the wedge-shaped transition section 360 and assumes the shape of the connecting structure 450, but not limited to, the letter "T", a hook, an anchor or a lug ring, as shown in Figs. 14a to 14d. The present invention is not limited to any specific shape of the connecting structure, as long as it can engage the recovery wire 850.

With additional reference to Figs. 15 and 13, in order to recover the stent, an engagement is established between the recovery wire 850 and the connecting structure 450 followed by fixation of the recovery wire 850. A recovery mechanism 650 is then pushed forward to advance a sheath 610 of the catheter until the stent is gradually contracted and loaded into the sheath 610. At last, all the components are retrieved from the human body.

### EMBODIMENT 4

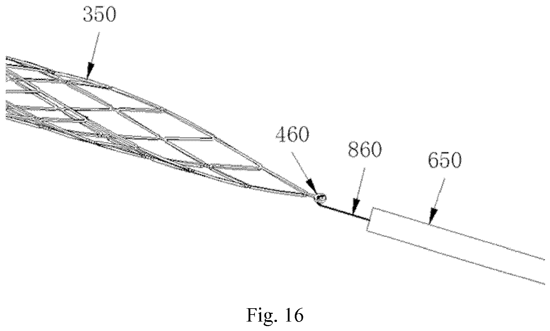

Referring to Figs. 16, a stent including a main body 350 and a connecting structure 460 is fabricated by laser cutting and thermally expanding a NiTi tube. The main body 350 is a mesh structure. The stent further includes a wedge-shaped mesh transition section 360 between the main body 350 and the connecting structure 460. The wedge-shaped transition section 360 has a mesh density gradually increasing along its length and can facilitate the compression and reception of the stent into a sheath of a catheter. The connecting structure 460 is a closed ring defined at one end of the wedge-shaped transition section 360.

A latex cover layer 100 is stitched onto the stent so that it is fixed inside the stent but does not cover the connecting structure 460 or the wedge-shaped transition section 360.

75 mg of paclitaxel and 45 mg of polyurea were dissolved in 2-4 ml of an acetone/ethanol mixture to obtain a solution, the solution was sprayed onto the surface of the cover layer and dried to obtain a stent-graft with a drug-containing coating 200.

Referring again to Fig. 12, the stent is chilled and compressed into the catheter 600, a push rod 700 is disposed in the catheter 600. The push rod 700 has a push-out head 500 at its one end proximal to the stent and is connected at the other end to connector of a stent delivery device. After the stent delivery device has delivered the stent to the site of a target lesion, it is slowly retracted with the push-out head 500 at the leading end of the push rod 700 bearing against the stent until the stent is released from the catheter 600 at the site of the target lesion.

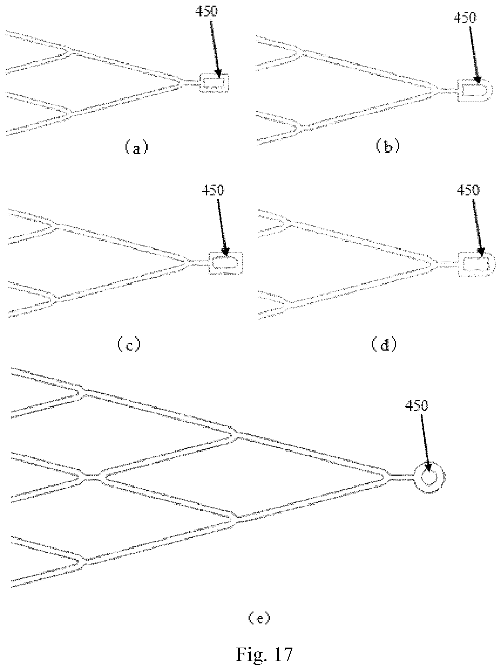

In this embodiment, the stent is detachable from a recovery wire 860. The delivery device and the push rod are retrieved from the human body, while the stent is left in the body. One end of the recovery wire 860 engages the connecting structure 460. The connecting structure 460 is defined at one end of the wedge-shaped transition section 360 and assumes the shape of the connecting structure 450 a closed ring which may be, but not limited to, a rectangular, semi-oblong or circular ring, as shown in Figs. 17a to 17e. The present invention is not limited to any specific shape of the connecting structure, as long as it can engage the recovery wire 860.

Referring again to Figs. 15 and 16, in order to recover the stent, an engagement is established between the recovery wire 860 and the connecting structure followed by fixation of the recovery wire 860. A recovery mechanism 650 is then pushed forward to advance a sheath 610 of the catheter until the stent is gradually contracted and loaded into the sheath 610. At last, all the components are retrieved from the human body.

In addition to the above described drug-applying method, the stent may include micropores and/or microgrooves, the drug may also be applied within micropores and/or microgrooves. The tubular body 300 may be braided from one or more metal wires or formed by cutting a metal tube. Preferably, a stent is a braided stent. The metal wire(s) or metal tube may be formed of a stainless steel, cobalt-chromium (CoCr) alloy, NiTi alloy, superelastic shape memory alloy or other material.

The cover layer 100 may be a perforated or braided PE, PTFE, PE, nylon, polyurethane, latex, silicone, bovine pericardium or porcine pericardium cover layer. The cover layer 100 may be sutured, sintered or electrostatically attached to an inner or outer surface of the tubular body.

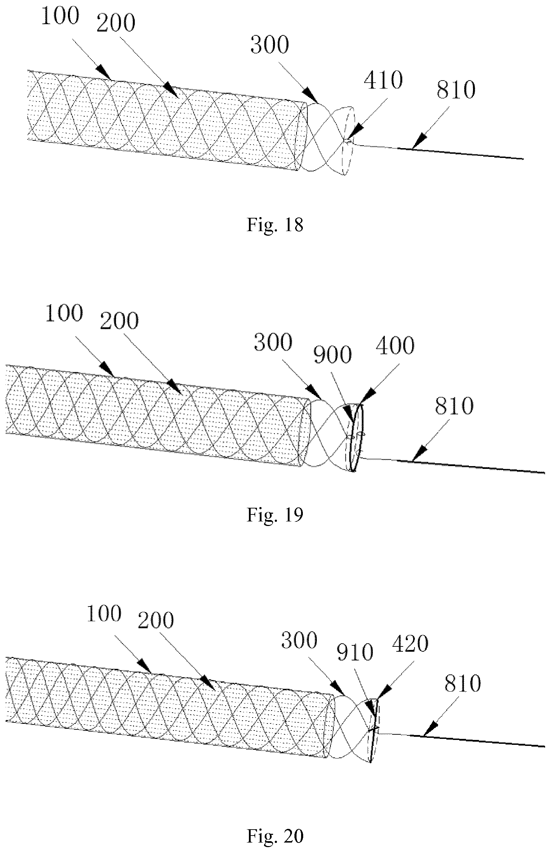

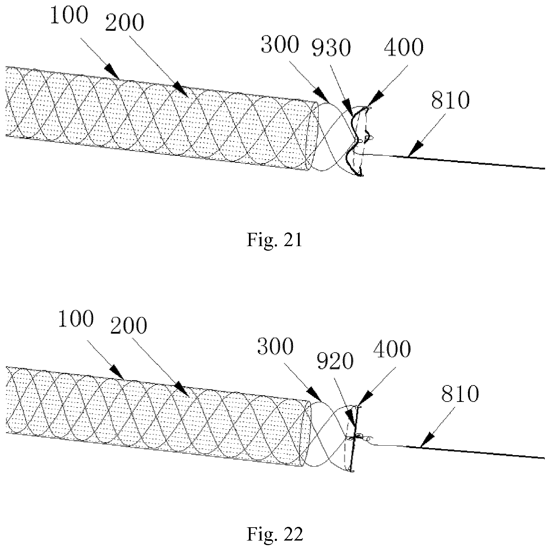

The connecting structure may be provided in one of a variety of different forms. For example, a lug ring 410 may be provided at one end of the connecting structure (Fig. 18). It is a matter of course that it may also be provided with more lug rings or hooks or not provided with any lug ring or hook. In order to facilitate the recovery, at the end of the connecting structure 400, there may be further provided an annular suture 900 (Fig. 19) or a crossed suture 910 (Fig. 20). Fig. 21 shows a variant suture 930 of the annular suture of Fig. 19. In this case, expect for the portions in contact with the connecting structure 400, the remainder of the suture 930 is attached to the outer surface of the stent beforehand. Further, as shown in Fig. 22, the connecting structure 420 may also be provided with a crossed and annular suture 920 at the intersection of the cross. During the recovery of the stent, the recovery wire may be hooked at one end with the suture.

The recovery wire may be a single wire leading from the connector of the stent delivery device, through the sheath and the connecting structure, and back to the connector. In case the recovery wire 800 is branched at one end, the branched end is connected to the connecting structure, and the other end is to the connector of the stent delivery device.

The connection between the recovery wire and the connecting structure of the stent may be detachable or undetachable connection accomplished by hooking, hitching, threading, welding, tying, etc.

The detachable connection is associated with, after the stent has been released in place, retrieving both the recovery wire and the stent delivery device from the human body and, during the recovery of the stent, inserting the recovery wire together with the stent delivery device into the human body, connecting it to the connecting structure, receiving the stent in the sheath and retrieving both the recovery wire and the stent delivery device from the human body.

The undetachable connection is associated with, after the stent has been released in place, leaving both the recovery wire and the stent delivery device in the human body and, during the recovery of the stent, retrieving all of them from the human body, or with, after the stent has been released in place, leaving the recovery wire in and retrieving the stent delivery device from the human body and, during the recovery of the stent, guiding the stent delivery device into the human body along the recovery wire, receiving the stent in the sheath and retrieving all of them.

The recoverability of the stent means that the stent can be recovered after it has stayed in the human body for a period of time of 5 minutes to 10 days, preferably 5 minutes to 1 day, more preferably 5 minutes to 7 hours.

The drug-containing coating 200 is comprised of a drug or of a drug and a hydrophilic additive. The drug may be an anti-proliferative, anti-inflammatory, antiphlogistic, antitumor, anti-mitotic, cytostatic, cytotoxic, anti-angiogenic, anti-restenotic, antimicrotubule, anti-metastatic or anti-thrombotic agent such as, for example, acemetacin, aescine, aminopterin, antimycoin, arsenic trioxide, aristolochic acid, aspirin, berberine, bilobol, rapamycin and its derivatives, paclitaxel, docetaxel, antibiotics (in particular actinomycin-D), hormones, anti-cancer antibodies, etc. The hydrophilic additive may be a water-soluble organic substance containing one or more functional groups selected from the group consisting of hydroxyl (-OH) groups, amino (-NH2) groups, amide (-CONH-) groups, sulfonic acid (-SO₃H) groups and carboxyl (-COOH) groups such as, for example, citric acid, dextran, pectin, vitamins, etc.

The drug-containing coating 200 may be prepared by dissolving the drug in an organic solvent and coating it on the cover layer through spraying the solution onto the cover layer or immersing the cover layer in the solution. The organic solvent may include, but not limited to, a mixture of water and one or more of methanol, ethanol, acetone, tetrahydrofuran, dimethylformamide, isopropanol, acetonitrile and ethyl acetate.

The drug-containing coating is configured to release 80% or more of the drug within a period of 15 seconds to 7 days, preferably 15 seconds to 1 day, more preferably 2 minutes to 6 hours since it comes into contact with the vessel wall.

In summary, the stent according to the present invention offer the benefits as follows: 1) it can support the vessel to prevent its contraction for a short period of time; 2) the short period of presence of the stent and cover layer ensures a sufficiently long time for the contact between the drug and vessel wall and hence good adsorption of the drug by the vessel; 3) its recoverability avoids the risk of damage to the human body from long-term presence of the stent; and 4) it allows reduced drug loss, increased drug absorption, a smaller amount of the used drug and lower toxicity to the human body.

The scope of the invention shall be as defined by the appended claims.

## Claims

1. A drug delivery device (1000, 1001) comprising a stent (1100, 1110) and a recovery mechanism (650), the stent (1100, 1110) comprising a tubular main body (300, 350), **characterized in that**:
the main body (300, 350) has a first end provided with a connecting structure (400, 420, 450, 460, 1111) configured for connection with the recovery mechanism (650); the main body (300, 350) has an inner or outer surface attached with a cover layer (100), the cover layer (100) coated with a drug; and the connecting structure (400, 420, 450, 460, 1111) is undetachably connected with the recovery mechanism (650),
the recovery mechanism (650) comprises a sheath (610, 1220, 1221) and a recovery wire (800, 850, 860, 1210, 1230) that passes through the sheath (610, 1220, 1221) and has one end connected to the connecting structure (400, 420, 450, 460, 1111) of the stent (1100, 1110), and the sheath (610, 1220, 1221) is configured to receive the stent (1100, 1110) when the stent (1100, 1110) is in a contracted configuration, the cover layer (100) is a bioabsorbable film.

2. The drug delivery device (1000, 1001) of claim 1, wherein the one end of the recovery wire (800, 850, 860, 1210, 1230) is fixedly connected to the connecting structure (400, 420, 450, 460, 1111) of the stent (1100, 1110).

3. The drug delivery device (1000, 1001) of claim 1 or 2, wherein the connecting structure (400, 420, 450, 460, 1111) comprises a plurality of connecting terminals; the one end of the recovery wire (800, 850, 860, 1210, 1230) defines a branched structure (1211, 1231) and each branch of the branched structure (1211, 1231) is configured to connect a corresponding one of the plurality of connecting terminals of the connecting structure (400, 420, 450, 460, 1111).

4. The drug delivery device (1000, 1001) of claim 1 or 2, wherein the connecting structure (400, 420, 450, 460, 1111) comprises at least two pairs of opposing connecting terminals connected to each other by a suture so that a cross is formed, and the recovery wire is configured to hook with the suture.

5. The drug delivery device (1000, 1001) of claim 1, wherein the main body (300, 350) is a mesh structure having a mesh density gradually increasing from the first end to a second end of the main body (300, 350), and the connecting structure (400, 420, 450, 460, 1111) is provided at the first end of the main body (300, 350) where the mesh density is lowest.

6. The drug delivery device (1000, 1001) of claim 1, wherein the drug comprises one or more of acemetacin, aescine, aminopterin, antimycoin, arsenic trioxide, aris-tolochic acid, aspirin, berberine, bilobol, rapamycin and derivatives thereof, paclitaxel, docetaxel, antibiotics and hormones.

7. The drug delivery device (1000, 1001) of claim 6, wherein a surface of the cover layer (100) is further applied with a hydrophilic additive which is a water-soluble organic substance containing one or more functional groups selected from the group consisting of hydroxyl groups, amino groups, amide groups, sulfonic acid groups and carboxyl groups.

## Patentansprüche

1. Medikamentenfreisetzungsvorrichtung (1000, 1001), umfassend einen Stent (1100, 1110) und einen Rückgewinnungsmechanismus (650), wobei der Stent (1100, 1110) einen röhrenförmigen Hauptkörper (300, 350) umfasst, **dadurch gekennzeichnet, dass**:
der Hauptkörper (300, 350) ein erstes Ende aufweist, das mit einer Verbindungsstruktur (400, 420, 450, 460, 1111) versehen ist, die zur Verbindung mit dem Rückgewinnungsmechanismus (650) ausgebildet ist; wobei der Hauptkörper (300, 350) eine mit einer Deckschicht (100) befestigte innere oder äußere Oberfläche aufweist, wobei die Deckschicht (100) mit einem Medikament beschichtet ist; und wobei die Verbindungsstruktur (400, 420, 450, 460, 1111) unlösbar mit dem Rückgewinnungsmechanismus (650) verbunden ist,
wobei der Rückgewinnungsmechanismus (650) eine Hülle (610, 1220, 1221) und einen Rückgewinnungsdraht (800, 850, 860, 1210, 1230) umfasst, der durch die Hülle (610, 1220, 1221) verläuft und ein Ende aufweist, welches mit der Verbindungsstruktur (400, 420, 450, 460, 1111) des Stents (1100, 1110) verbunden ist, und wobei die Hülle (610, 1220, 1221) ausgebildet ist, den Stent (1100, 1110) aufzunehmen, wenn der Stent (1100, 1110) in einer kontrahierten Anordnung ist, wobei die Deckschicht (100) eine bioresorbierbare Folie ist

2. Medikamentenfreisetzungsvorrichtung (1000, 1001) nach Anspruch 1, wobei das eine Ende des Rückgewinnungsdrahts (800, 850, 860, 1210, 1230) fest mit der Verbindungsstruktur (400, 420, 450, 460, 1111) des Stents (1100, 1110) verbunden ist.

3. Medikamentenfreisetzungsvorrichtung (1000, 1001) nach Anspruch 1 oder 2, wobei die Verbindungsstruktur (400, 420, 450, 460, 1111) eine Vielzahl von Verbindungsanschlüssen umfasst; wobei das eine Ende des Rückgewinnungsdrahts (800, 850, 860, 1210, 1230) eine verzweigte Struktur (1211, 1231) definiert und jeder Zweig der verzweigten Struktur (1211, 1231) ausgebildet ist, jeweilig einen der Vielzahl von Verbindungsanschlüssen der Verbindungsstruktur (400, 420, 450, 460, 1111) zu verbinden.

4. Medikamentenfreisetzungsvorrichtung (1000, 1001) nach Anspruch 1 oder 2, wobei die Verbindungsstruktur (400, 420, 450, 460, 1111) mindestens zwei Paare von gegenüberliegenden Verbindungsanschlüssen umfasst, die durch eine Naht miteinander verbunden sind, so dass ein Kreuz gebildet wird, und wobei der Rückgewinnungsdraht ausgebildet ist, in das Nahtmaterial ein zu haken.

5. Medikamentenfreisetzungsvorrichtung (1000, 1001) nach Anspruch 1, wobei der Hauptkörper (300, 350) eine Maschenstruktur mit einer Maschendichte ist, die vom ersten Ende zu einem zweiten Ende des Hauptkörpers (300, 350) sukzessive zunimmt, und wobei die Verbindungsstruktur (400, 420, 450, 460, 1111) am ersten Ende des Hauptkörpers (300, 350), an dem die Maschendichte am niedrigsten ist, vorgesehen ist.

6. Medikamentenfreisetzungsvorrichtung (1000, 1001) nach Anspruch 1, wobei das Medikament eines oder mehrere von Acemetacin, Aescin, Aminopterin, Antimycoin, Arsentrioxid, Aris-Tolochinsäure, Aspirin, Berberin, Bilobol, Rapamycin und Derivate davon, Paclitaxel, Docetaxel, Antibiotika und Hormone umfasst.

7. Medikamentenfreisetzungsvorrichtung (1000, 1001) nach Anspruch 6, wobei eine Oberfläche der Deckschicht (100) ferner mit einem hydrophilen Additiv beaufschlagt ist, das eine wasserlösliche organische Substanz ist, die eine oder mehrere funktionelle Gruppen, ausgewählt aus der Gruppe bestehend aus Hydroxylgruppen, Aminogruppen, Amidgruppen, Sulfonsäuregruppen und Carboxylgruppen, enthält.

## Revendications

1. Dispositif d'administration de médicament (1000, 1001) comprenant un stent (1100, 1110) et un mécanisme de récupération (650), le stent (1100, 1110) comprenant un corps tubulaire principal (300, 350), **caractérisé en ce que** :
le corps principal (300, 350) possède une première extrémité dotée d'une structure de connexion (400, 420, 450, 460, 1111) conçue pour une connexion avec le mécanisme de récupération (650) ; le corps principal (300, 350) possède une surface interne ou externe fixée à une couche de recouvrement (100), la couche de recouvrement (100) étant revêtue d'un médicament ; et la structure de connexion (400, 420, 450, 460, 1111) est connectée de façon non détachable avec le mécanisme de récupération (650),
le mécanisme de récupération (650) comprend une gaine (610, 1220, 1221) et un fil de récupération (800, 850, 860, 1210, 1230) qui passe à travers la gaine (610, 1220, 1221) et a une extrémité connectée à la structure de connexion (400, 420, 450, 460, 1111) du stent (1100, 1110), et la gaine (610, 1220, 1221) est conçue pour recevoir le stent (1100, 1110) quand le stent (1100, 1110) est dans une configuration contractée, la couche de recouvrement (100) est un film biorésorbable.

2. Dispositif d'administration de médicament (1000, 1001) selon la revendication 1, dans lequel l'extrémité du fil de récupération (800, 850, 860, 1210, 1230) est connectée à demeure à la structure de connexion (400, 420, 450, 460, 1111) du stent (1100, 1110).

3. Dispositif d'administration de médicament (1000, 1001) selon la revendication 1 ou 2, dans lequel la structure de connexion (400, 420, 450, 460, 1111) comprend une pluralité de bornes de connexion; l'extrémité du fil de récupération (800, 850, 860, 1210, 1230) définit une structure ramifiée (1211, 1231) et chaque branche de la structure ramifiée (1211, 1231) est configurée pour connecter une borne correspondante de la pluralité de bornes de connexion de la structure de connexion (400, 420, 450, 460, 1111).

4. Dispositif d'administration de médicament (1000, 1001) selon la revendication 1 ou 2, dans lequel la structure de connexion (400, 420, 450, 460, 1111) comprend au moins deux paires de bornes de connexion opposées connectées entre elles par une suture de sorte qu'une croix soit formée, et le fil de récupération est conçu pour s'accrocher à la suture.

5. Dispositif d'administration de médicament (1000, 1001) selon la revendication 1, dans lequel le corps principal (300, 350) est une structure à mailles possédant une densité de mailles augmentant progressivement depuis la première extrémité vers une seconde extrémité du corps principal (300, 350), et la structure de connexion (400, 420, 450, 460, 1111) est prévue au niveau de la première extrémité du corps principal (300, 350) là où la densité de mailles est la plus faible.

6. Dispositif d'administration de médicament (1000, 1001) selon la revendication 1, dans lequel le médicament comprend un ou plusieurs parmi l'acétamicine, l'escine, l'aminoptérine, l'antimycoïne, le trioxyde d'arsenic, l'acide aristolochique, l'aspirine, la berbérine, le bilobol, la rapamycine et des dérivés de ceux-ci, le paclitaxel, le docétaxel, des antibiotiques et des hormones.

7. Dispositif d'administration de médicament (1000, 1001) selon la revendication 6, dans lequel une surface de la couche de recouvrement (100) est en outre appliquée avec un additif hydrophile qui est une substance organique hydrosoluble contenant un ou plusieurs groupes fonctionnels choisis dans le groupe constitué par les groupes hydroxyle, les groupes amino, les groupes amide, les groupes acide sulfonique et les groupes carboxyle.
